# EUROPEAN PATENT SPECIFICATION

(11) **EP 3 615 655 B1**
(45) Date of publication and mention of the grant of the patent: **04.12.2024**
(21) Application number: 18726854.5
(22) Date of filing: 19.04.2018
(51) Int. Cl.: C12M 1/32, C12M 1/42, G01N 3/32

(54) **RHYTHMIC CELL STAMPER AND THE APPLICATION THEREOF**
RHYTHMISCHER ZELLENSTAMPFER UND ANWENDUNG DAVON
MATRICE DE PRESSAGE DE CELLULES RYTHMIQUES ET SON APPLICATION

(30) Priority: 24.04.2017 HU 1700174
(43) Date of publication of application: 04.03.2020
(73) Proprietor: Sasvari, Csaba, 4034 Debrecen (HU)
(72) Inventor: Sasvari, Csaba, 4034 Debrecen (HU)
(74) Representative: Pintz, György
(86) International application number: PCT/IB2018/052713
(87) International publication number: WO 2018/197998

(56) References cited:
- WO-A1-2015/069890
- US-A- 4 940 853
- US-A1- 2015 017 719

## Description

The subject of the invention is a rhythmic cell stamper for laboratory use, and the procedure for the application of the apparatus.

The disorders of the soft tissues of the skeletal system (e.g. cartilage, tendon, ligament, joint capsules, etc.) are often initiated by the mechanical load exceeding optimum levels, therefore it is of essential importance to know how the mechanical stimulus affecting the cells and tissues produces cell physiology impacts, i.e. how mechanotransduction takes place.

Another example, regarding the effect of the mechanical stimulus on the tissues of a certain organ or several organs, and the effect on their functioning, is the circulatory system. The myocardium tissue, the epithelial cells of the vascular system (endothelial cells), and particularly the smooth cells located in the wall of blood vessels and especially in the arteries are both sensitive to the velocity of the blood flowing in the lumen and the mechanical stimulus induced by hydrostatic pressure.

Similarly, the type, the intensity and frequency of the mechanical stimuli affecting the cells heavily influences the direction of development of undifferentiated cells. The effect of mechanical stimuli from the environment on cells and tissues is not only significant under physiological conditions, but also the biological properties of malignant cell proliferation can be substantially influenced by the mechanical effects arising from the tumor environments.

Among research scientists, there is a significant need to provide adequate and reproducible research conditions to mimic the natural mechanical effects influencing cells and tissues for study purposes. Namely, to find out through what molecular mechanisms they influence the most diverse biological characteristics of cells, tissues and molecules.

The state of the art includes the following solutions.

Many people are looking for solutions assisting research labs in the field of mechanotransduction (mechanical stimuli effecting cells and tissues). In order to satisfy such need, many research laboratories use self-developed, small scale crafted laboratory tools especially made for the given experiment.

In some systems, the touch by the pipette tip produces the physical stimulus on the propagated cells and tissue, which is obviously an intervention characterized by uncertain physical parameters, which can not be practically reproduced even under the same experimental conditions.

Also, a procedure is employed when liquid droplets land on the cells, tissues or on the biological carriers including them (e.g. alginate droplets) that are to be stimulated. Another known method is when the cartilage cells encapsulated in collagen or alginate droplets are stimulated with the direct compression of the microscopic droplets.

There are several national and international doctoral thesis and studies available about the procedures applying the alginate droplets.

The disadvantage of these solutions is that the alginate or collagen droplet technique is primarily suitable for studying mature cartilage cells, however, the response on the mechanical stimulus given by other, adherent culturable cells, such as bone cells, muscle cells, connective tissue cells, epithelial cells or even undifferentiated stem cells, adherent tumor cells, or differentiating cell cultures cannot be studied in this way. The reproduction possibility of this research is very low.

Several industrial companies manufacture and distribute devices and equipment allowing cell and tissue level studying of mechanical stimuli. Some of these devices are to meet specific needs, while other devices offer versatile functions.

The instrument described on http://www.tainstruments.com/3dculturepro-bioreactors/ simulates the effects of mechanical stimuli. This allows the examination of tissues (bone, cartilage, vascular constructs, skin, ligaments and tendons) in in-vitro settings.

The disadvantage of the above equipment is that the use of the popular, 6-well cell culture plates is not possible, however, these are the carriers of culturing and then testing the living cultures in the culture media. The device of the mentioned manufacturer allows research options only by using the research frames provided for their own instrument.

The equipment described on http://www.tainstruments.com/5200-products/ is also able to simulate the impact of mechanical stimulus. It contains versatile chambers adaptable to blood vessels, cardiac muscle, bone, cartilage, meniscus, spinal discs, ligaments, tendons, and skin. The independent operation of the multi-chamber system allows the controlled loading of the samples. Multi-axial loading capabilities and characterization of mechanical properties can be simulated. The disadvantage of this solution is that it is mainly based on tensile and torsion forces, where the tension force is communicated with the cells/tissues by adhering them to a biocompatible flexible base (e.g. collagen or polymer membrane), and by pulling or torsioning this base, the physical stimulus is achieved. Another disadvantage is that the size of the equipment inhibits the use of commonly used sterile thermostats. The document US 4940853 A describes a device suitable to apply compression on a cell culture using a compression head.

The object of this invention is to overcome the problems of the present solutions and to provide an apparatus and a procedure capable of producing such research, examination and technical possibilities for the stimulation of cells, tissues and molecules, which can be documented, reproduced, furthermore allows the use of popular tools, and which is also mobile and compatible with other in-vitro environmental researches. In addition, the stimulus can be applied gently, to maintain the integrity of the vulnerable cultures.

The inventive step is based on the recognition that an invention, which is more advantageous than the previous ones, may be created by executing the procedure according to claim 1.

Part of the invention is the recognition of the need for a procedure, which is based on a novel technological basis for the application in cell and tissue culture and in molecular research, and the implementation of a research laboratory solution. Using our automated mechanical stamper mechanism, the desired mechanical stimulation can be realised. All this takes place through a controlled and computerized automated research process, offering reproducible research with identical results.

Part of the invention is the recognition that multiple workflows, simultaneous stamping of multiple types of culture are needed, and we have created a solution that provides the stimulus for the experimental substances taken directly from the maintenance thermostats. All this while maintaining the stimulus of the live cultures over several days, because during the workflow, nutrient solutions can be added freely, without stopping the running of the program. Furthermore, part of the invention is the recognition that we need to adapt our instrument to the size of the most common thermostats, and the fact that it should be relocated easily, but at the same time should also provide precision operation afterwards. In addition, it ensures more precise control and stamping of the stimulation points, therefore there is a need for such solution, which stimulates only the fluid flow or the carrier media, thereby being gentle to the culture. The new generation rhythmic cell stamper instrument corresponds to this recognized need of research. According to our goal, the most general implementation form of the invention is described in claim 1. The most general form of the application procedure is described in the independent claim 6. The various implementation forms are described in the dependent claims.

The solution is a general rhythmic cell stamper instrument for simultaneous stimulation of the cells, cell cultures and molecules adhering to a bottom of a 6-well Cell Culture Plate, the instrument comprising of a Base, at least one Support Leg, at least one Compression Head, Casing and a Drive Unit connected to the Compression Head; and a Computer connected to the Drive Unit. The feature of the invention is that it comprises of a Release Fixing Mechanism for allowing the unmounting and remounting of the at least one Compression Head manually; a Computer for setting the time, length and force of mechanotransduction and recording the set data; a Gyroscope connected to the Computer and/or Radiofrequency Communication Unit; at least one Measuring Rod for controlling the path of the at least one Compression Head; and a built-in Controller and Memory Unit; wherein the Instrument is equipped with Plates containing culture fluid, the instructions of the software in the computer allow to maintain the at least one Compression Head in contact with the culture fluid within the Plates, and the Compression Head is configured to mechanically stimulate the culture fluid located in the Plates partly via a force transmitted by the culture fluid and partly by a fluid flow generated by the at least one Compression Head moving in the culture fluid.

According to the different versions, the drive unit can be an electromagnetic and/or stepping motor and the power supply of the drive unit can be provided by an external power source and/or internal battery.

In another design we could connect a stepping motor to the support leg.

In another example of the versions, the compression head and/or the release fixing mechanism and/or the casing can be made of stainless steel.

In another option it is equipped with three Plates, and an automatic leveling system for keeping the Instrument and the Plates in a horizontal position.

Part of the invention is the procedure of the application of the rhythmic cell stamper. The feature of the application is that the time, distance and strength of the mechanotransduction are set on the computer and the set data are recorded; the compression head is moved by the drive unit; the measurement is repeated, reproduced and checked with the recorded data.

The procedure can be implemented also while the instrument is kept horizontally.

The procedure can be implemented also by manually unmounting and remounting the release fixing mechanism manually for replacing the compression head.

The procedure can be implemented including the step of stimulating different cultures on the three Plates (5) with three different programs simultaneously, with different frequencies, in a mechanically reproducible way.

The invention is presented in more detail using drawings relating to its implementation forms.

Among the attached drawings,

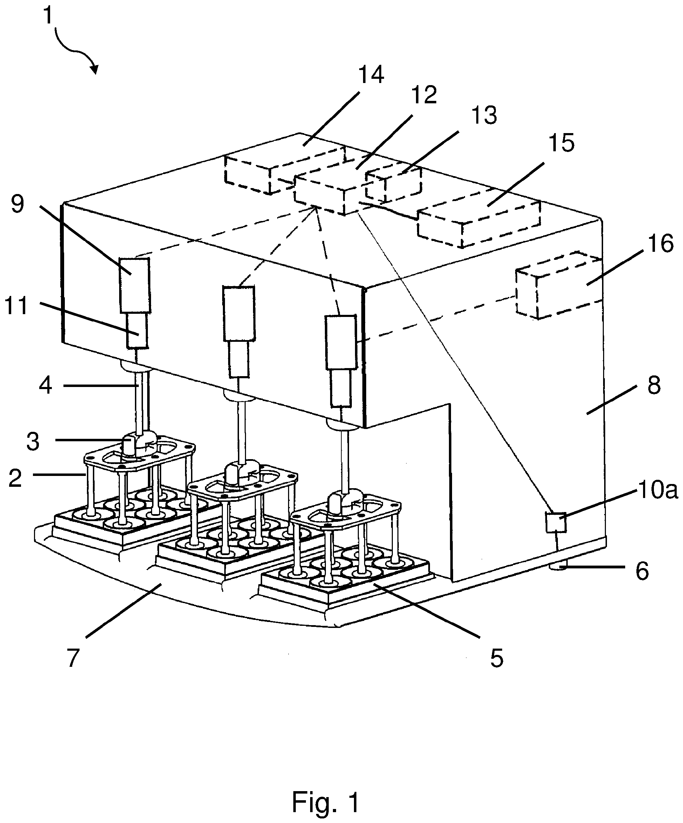

Figure 1 illustrates the axonometric drawing of the instrument.

Figure 1 illustrates the construction of a new generation rhythmic cell stamping Instrument 1, which provides reproducible research results for laboratories, in respect of testing responses and changes in cells, tissues and molecules to mechanical loads under controlled conditions. In Instrument 1, the physical stimulus cycle and the force parameters can be manually adjusted, and also by the Computer 12 software within the Instrument 1. The operating parameters of the Instrument 1 can be controlled entirely externally, for example, via the Internet, in remote desktop function, by means of an external computer, which is connected to the Radio Frequency Communication Unit 14 within Instrument 1. Such operating parameters include, for example, the time, distance and power of the mechanotransduction, which data is also recorded by Computer 12, therefore the research results can be reproduced and checked everywhere and any time. Instrument 1 is equipped with a built-in Controller and Memory Unit 13, which allows Instrument 1 to independently implement the work instructions or the research program, so there is no need of an online connection with an external computer. With the software, the automated documentation and strict tracking of experiments, computerized recording of research data, statistical analysis, registration of the operational technical analytics, and registration of required automatic level adjustments are all possible.

Instrument 1 is also suitable for studying the effects of the cyclic compression forces in the physiological magnitude, for example on the life of the cells, under laboratory conditions. Instrument 1 allows the simultaneous stimulation of the cells, cell cultures and molecules adhering to the bottom of the 6-well Cell Culture Plate 5, widely used in cell experiments.

The reproduction of the experimental results is ensured by the fact that Compression Head 2 performs a mechanotransduction path, with its distance specified already during manufacture. Compression Heads 2 are moved by Drive Unit 9 located in the housing of Instrument 1 through Holding Rods 4, where Drive Unit 9 can be a Stepping Motor 110a and/or an electromagnet. The transmission of the mechanical stimulus generated by Instrument 1 is partly represented by the force transmitted by culture fluid and partly by the fluid flow generated by Compession Head 2, moving in the fluid. The contact surface of Compression Heads 2 is concave, planar, convex or polygonal, depending on the type of compression force or rheologic factor required by the mechanical stamping. Both mechanical stimulus types typically simulate the physical forces occurring in cells, tissues and molecules during normal physical activity, such as the physical forces affecting the cartilage surface of the joints. In case of Stepping Motor 10a implementation, the movement of Compression Heads 2 can be set, i.e. the length, time and force of the mechanical stimulus can be specified. These parameters can be measured, saved and reproduced during the experiments, and the real time implementation of such parameters are confirmed by Instrument 1. By moving the transducer Compression Head 2 driven by the Stepper Motor 110a, such as by moving down/up in fluid, besides the compression effect the decompression effect can also be applied to the cell, tissue or molecular cultures.

In the electromagnetic design, when manufacturing Instrument 1, the electromagnet is installed and fabricated by means of a specified and fixed mechanotransduction stamping pathway. The path of Holding Rod 4, transmitting the mechanical force, is physically limited by the distance capacity of the pusher after the electromagnetic voltage. The unaltered distance ensures the reproducibility of the experiment. The stimulation force can be set by precise metering of the transfer medium (e.g. by pipette), or the force can be also set by the same amount of cell, tissue or molecule culture weight.

For both stepping motors and electromagnetic drive, Measuring Rods 11 for controlling the paths of the Compression Heads 2 are incorporated in Instrument 1. The electronic Measuring Rod 11 indicates the starting and ending points of the holding rods with the accuracy of 0.01 mm, thereby the stimulation data of particular cell, tissue or molecular cultures can be reproduced and controlled.

Release Fixing Mechanism 3 ensures the replacing of Compression Heads 2 implementing the direct stimulation manually. This allows the unmounting and remounting of Compression Heads 2 without any tools, which are responsible for the mechanical stimulation of the research substance located in Plates 5. The complete construction of Compression Heads 2 and the Release Fixing Mechanism 3 is made of stainless steel, of the so called surgical steel. This ensures multiple usability, in addition to multiple sterilizations, and the calibrated size design during manufacture. The positioning of the contact points Compression Heads 2 in a single plane must be ensured at all six points of Plates 5. When replacing Plates 5 containing the research substance, the replacement of the Compression Heads 2 on the given holding rods is also required. This replacement shall be carried out as fast as possible in the in-vitro and/or sterile environments, and this is provided by the quick Release Fixing Mechanism 3.

In Instrument 1 three Plates 5 can be placed beneficially, and to ensure identical stimulus, shall be kept horizontally. Base 7 under Plates 5 is placed at the calibrated same distance from Compression Heads 2 during manufacturing, to ensure the same, identical stimulation of the research substances. For the purpose of level correction during operation, Instrument 1 has an automatic leveling system which keeps Instrument 1 and Plates 5 in a continuous horizontal position. Instrument 1 has three bearing points, of which the two Support Legs 6 below the rear part of Instrument 1 can be levelled with the connected independent Stepping Motors 10a. Part of the automatic leveling system is a three-axis Gyroscope 15 that continuously measures the Earth's magnetic field and calculates the measured deviation from the horizontal level, and their X, Y, Z coordinates, and then provides this data to the Controller and Memory Unit 12 of Computer 12. Computer 12 records the measured values and deviations in the research protocol, and in case of deviation, Control and Memory Unit 13 instructs Stepping Motors 10a connected to Support Legs 6 to move Instrument 1 horizontally by moving Stepping Motors 10a. Since Instrument 1 is mobile and can be easily transferred to another thermostat (carbon-dioxide thermostat), continuous horizontal level monitoring may be necessary.

Due to the better simulation of the human body, the in-vitro environment, cell stimulation is often carried out in a carbon-dioxide thermostat. The carbon-dioxide thermostats are characterized by high humidity exceeding 90% volume, and by carbon-dioxide content over 5% volume, as well as thirty-seven degrees of research medium. In such a medium, many substances may deform, resulting in distortion of experimental results. As expected, Instrument 1 is fitted with a stainless steel and vapor barrier outer Casing 8, and Compression Heads 2 and Release Fixing Mechanism 3 are made of stainless material, which ensures long service life and reliable operation even against the aggressive corrosive media of the carbon-dioxide thermostats.

The in-vitro environment applied in the research and the possible sterility are maintained with the Radio Frequency Communication Unit 14. Mechanical stimulation of cells, tissues and molecules, i.e. the mechanotransduction does not actually allow the termination of continuous operation. For lifelike simulation and accurate results, it is necessary to maintain continuous and controlled workflows. In case of the external control of Instrument 1, a solution is therefore required to withstand any possible aggressive environment, which is a carbon-dioxide thermostat. The Radio Frequency Communication Units 14 used in the present invention are freely usable, but are also equipped with adequate data protection, so it is ensured that Instrument 1 can be controlled externally and the research materials and results are not compromised during the process. Therefore there is no need for data cables, data carriers, control units and control feedback units, so it is also possible to maintain a sterile working environment within the carbon dioxide thermostat, which is a primary factor for the laboratories.

Another solution contributing to the sterile working environment is the fact that it is not necessary to connect an external electrical power source to Instrument 1 to ensure permanent mechanical stamping, instead, Instrument 1 may also be equipped with Internal Batteries 16. During the running of a regular experiment program, the Batteries 16 can operate Instrument 1 reliably for a period of weeks or even a month. Such operating time is long enough implementing the durable mechanical tests of cells, tissues and molecules in a sterile, fully enclosed in-vitro environment. Instrument 1 therefore complies with the expectations towards the research laboratory equipment, in terms of maintenance, stable and independent operation of the in-vitro medium from environmental elements.

Instrument 1 is automatic and is suitable for intermittent, uniaxial mechanical stamping of cells, tissues and molecules. With its use, reliable operation in the carbon-dioxide thermostats used for cell and tissue culturing, without using external power supply for a period of weeks or even a month can be used, for example with Internal Batteries 16, can be achieved. The operation of Instrument 1, based on external computer control, is accomplished by means of radio frequency communication for the purpose of maintaining the sterility of the carbon-dioxide thermostat, via the 14 Radio Frequency Communication Units. Thanks to the radio frequency communication, there is no need for data carriers, control units and control feedback units. The intervention parameters can be documented automatically, with a computer, by Instrument 1.

The invention introduced has several advantages. One advantage of the invention is that it is capable of stimulating the different cultures of the three separates plates with three different programs simultaneously, with different frequencies, in a mechanically reproducible way.

The new generation rhythmic cell stampers can provide users with new innovative solutions for laboratory technology for researchers or pharmaceutical companies. Among other things, research laboratories of pharmaceutical companies producing preparations for the healing of the skeletal system or maintaining it, can take advantage of it. The equipment facilitates their in-vitro testing. Since especially the diseases of the soft tissues of the skeletal system (such as cartilage, tendon, ligaments, joints, etc.) are very often caused by mechanical loads exceeding the optimum, it is therefore extremely important to know how the mechanical stimulus of the cells and tissues influence the cell physiological effects, i.e. how mechanotransduction takes place. At the same time, the opposite role of mechanical load can be studied, as well, namely, when the mechanical load is not achieving the optimum degree or is completely missing, the components of the skeleton system get weak and result in tissue degradation. (for example: space research)

The new generation rhythmic cell stamper is primarily designed and optimized for use in adherent cell and tissue cultures, but the possibility to modify the distance of the movement of the compression heads also makes it possible to expose cells, tissues and molecules integrated into flexible or more rigid "scaffold" (carrier) to compression force by direct contact with the compression head.

Using the new generation rhythmic cell stamping instrument, scientists can obtain a more accurate picture of the amount of physical load necessary to make the support the optimum structure of the skeletal system tissues. The instrument models the effects of certain physical therapy interventions on cartilage and bone tissue in laboratory conditions, and in the longer term the experimental results may provide extrapolation information for therapy.

The use of the invention is widely applicable, it is suitable for modeling any process where mechanical stimuli from the environment play a significant effect on cell metabolism, cell division, etc.

The most important feature of the invention for the researchers is the mode, ensuring the reproducibility and controllability of the operational parameters, i.e. the experimental conditions. Because well-structured, reliably stored data can be accessed at any time and from any location - by the properly authorized users -, the research and experiment results can be reproduced and verified.

Another advantage is that the instrument can be applied universally due to its size fitting the world's most widely used carbon-dioxide thermostats.

The primary field of application of the invention is cell and tissue culture and molecular research, especially laboratory tests where mechanical stamping of cells, tissues, molecules and its reproduction are required.

## Claims

1. Rhythmic cell stamper instrument (1) for simultaneous stimulation of the cells, cell cultures and molecules adhering to a bottom of a 6-well Cell Culture Plate (5), the instrument (1) comprising of a Base (7), at least one Support Leg (6), at least one Compression Head (2), Casing (8) and a Drive Unit (9) connected to the Compression Head (2); and a Computer (12) connected to the Drive Unit (9), **characterized in that** the Instrument (1) further includes a Release Fixing Mechanism (3) for allowing the unmounting and remounting of the at least one Compression Head (2) manually; a Computer (12) for setting the time, length and force of mechanotransduction and recording the set data; a Gyroscope (15) connected to the Computer (12) and/or Radiofrequency Communication Unit (14); at least one Measuring Rod (11) for controlling the path of the at least one Compression Head (2); and a built-in Controller and Memory Unit (13); wherein the Instrument (1) is equipped with Plates (5) containing culture fluid,
the instructions of the software in the computer allow to maintain the at least one Compression Head (2) in contact with the culture fluid within the Plates (5), and the Compression Head is configured to
mechanically stimulate the culture fluid located in the Plates (5) partly via a force transmitted by the culture fluid and partly by a fluid flow generated by the at least one Compression Head (2) moving in the culture fluid.

2. The instrument according to claim 1, **characterized in that** the Drive Unit (9) is an electromagnet and/or a Stepping Motor (10a) and the power supply of the Drive Unit (9) is an external power source and/or an Internal Battery (16).

3. The instrument according to claim 1 or 2, **characterized in that** there is a Stepping Motor (10a) connected to the Support Leg (6).

4. The instrument according to any of claims 1 to 3, **characterized in that** the Compression Head (2) and/or the Release Fixing Mechanism (3) and/or the Casing (8) are made of stainless steel.

5. The instrument according to any of claims 1 to 3, **characterized in that** it is equipped with three Plates (5), and an automatic leveling system for keeping the Instrument (1) and the Plates (5) in a horizontal position.

6. Procedure for the application of the instrument according to claim 1, **characterized in that** the time, distance and strength of the mechanotransduction are set on the Computer (12) and the set data are recorded; the Compression Head (2) is moved by the Drive Unit (9); the measurement is repeated, reproduced and checked with the recorded data.

7. The procedure according to claim 6, **characterized in that** during the measurement, Instrument (1) shall be kept horizontally.

8. The procedure according to claim 6 or 7, **characterized in that** when replacing the compression head (2) the release fixing mechanism (3) is unmounted and then remounted manually.

9. The procedure according to any of claims 6 to 8, **characterized in that** it also includes the step of stimulating different cultures on the three Plates (5) with three different programs simultaneously, with different frequencies, in a mechanically reproducible way.

## Patentansprüche

1. Rhythmisches Zellstempelinstrument (1) zur gleichzeitigen Stimulation der Zellen, Zellkulturen und Moleküle, die am Boden einer 6-Well-Zellkulturplatte (5) haften, wobei das Instrument (1) eine Basis (7), mindestens ein Stützbein (6), mindestens einen Kompressionskopf (2), ein Gehäuse (8) und eine mit dem Kompressionskopf (2) verbundene Antriebseinheit (9) umfasst; und einen mit der Antriebseinheit (9) verbundenen Computer (12), **dadurch gekennzeichnet, dass** das Instrument (1) ferner einen Lösungsbefestigungsmechanismus (3) zum manuellen Abmontieren und Wiederanbringen des mindestens einen Kompressionskopfes (2) aufweist; einen Computer (12) zum Einstellen der Zeit, Länge und Kraft der Mechanotransduktion und zum Aufzeichnen der eingestellten Daten; ein Gyroskop (15), das mit dem Computer (12) und/oder der Funkfrequenz-Kommunikationseinheit (14) verbunden ist; mindestens eine Messstange (11) zur Steuerung des Weges des mindestens einen Kompressionskopfes (2); und eine eingebaute Steuerungs- und Speichereinheit (13); wobei das Instrument (1) mit Platten (5) ausgestattet ist, die Kulturflüssigkeit enthalten,
die Anweisungen der Software im Computer ermöglichen es, den mindestens einen Kompressionskopf (2) in Kontakt mit der Kulturflüssigkeit innerhalb der Platten (5) zu halten, und der Kompressionskopf ist so konfiguriert, dass er
die in den Platten (5) befindliche Kulturflüssigkeit teilweise über eine durch die Kulturflüssigkeit übertragene Kraft und teilweise durch eine Flüssigkeitsströmung, die durch den sich in der Kulturflüssigkeit bewegenden mindestens einen Kompressionskopf (2) erzeugt wird, mechanisch stimuliert.

2. Instrument nach Anspruch 1, **dadurch gekennzeichnet, dass** die Antriebseinheit (9) ein Elektromagnet und/oder ein Schrittmotor (10a) ist
und die Stromversorgung der Antriebseinheit (9) eine
externe Stromquelle und/oder eine interne Batterie (16) ist.

3. Instrument nach Anspruch 1 oder 2, **dadurch gekennzeichnet, dass** ein mit dem Stützbein (6) verbundener Schrittmotor (10a) vorhanden ist.

4. Instrument nach einem der Ansprüche 1 bis 3, **dadurch gekennzeichnet, dass** der Kompressionskopf (2) und/oder der Lösungsbefestigungsmechanismus (3) und/oder das Gehäuse (8) aus Edelstahl bestehen.

5. Instrument nach einem der Ansprüche 1 bis 3, **dadurch gekennzeichnet, dass** es mit drei Platten (5) und einem automatischen Nivellierungssystem ausgestattet ist, um das Instrument (1) und die Platten (5) in einer horizontalen Position zu halten.

6. Verfahren zur Anwendung des Instruments nach Anspruch 1, **dadurch gekennzeichnet, dass** die Zeit, der Abstand und die Stärke der Mechanotransduktion am Computer (12) eingestellt und die eingestellten Daten aufgezeichnet werden; der Kompressionskopf (2) durch die Antriebseinheit (9) bewegt wird; die Messung mit den aufgezeichneten Daten wiederholt, reproduziert und überprüft wird.

7. Verfahren nach Anspruch 6, **dadurch gekennzeichnet, dass** während der Messung das Instrument (1) horizontal gehalten wird.

8. Verfahren nach Anspruch 6 oder 7, **dadurch gekennzeichnet, dass** beim Austausch des Kompressionskopfes (2) der Lösungsbefestigungsmechanismus (3) manuell abmontiert und dann wieder angebracht wird.

9. Verfahren nach einem der Ansprüche 6 bis 8, **dadurch gekennzeichnet, dass** es auch den Schritt des gleichzeitigen Stimulierens verschiedener Kulturen auf den drei Platten (5) mit drei verschiedenen Programmen, mit unterschiedlichen Frequenzen, auf mechanisch reproduzierbare Weise beinhaltet.

## Revendications

1. Instrument de stimulation rythmique des cellules (1) pour la stimulation simultanée des cellules, des cultures cellulaires et des molécules adhérant au fond d'une plaque de culture cellulaire à 6 puits (5), l'instrument (1) comprenant une base (7), au moins un pied de support (6), au moins une tête de compression (2), un boîtier (8)
et une unité d'entraînement (9) reliée à la tête de compression (2) ; et un ordinateur (12) relié à l'unité d'entraînement (9), **caractérisé en ce que** l'instrument (1) comprend en outre un mécanisme de fixation de libération (3) permettant le démontage et le remontage manuel de la ou des têtes de compression (2) ; un ordinateur (12) pour régler le temps, la longueur et la force de la mécanotransduction et enregistrer les données définies ; un gyroscope (15) relié à l'ordinateur (12) et/ou à l'unité de communication radiofréquence (14) ; au moins une tige de mesure (11) pour contrôler le trajet de la ou des têtes de compression (2) ; et une unité de contrôle et de mémoire intégrée (13) ; dans lequel l'instrument (1) est équipé de plaques (5) contenant du fluide de culture, les instructions du logiciel dans l'ordinateur permettent de maintenir la ou les têtes de compression (2) en contact avec le fluide de culture dans les plaques (5), et la tête de compression est configurée pour stimuler mécaniquement le fluide de culture situé dans les plaques (5) en partie via une force transmise par le fluide de culture et en partie par un écoulement de fluide généré par la ou les têtes de compression (2) se déplaçant dans le fluide de culture.

2. Instrument selon la revendication 1, **caractérisé en ce que** l'unité d'entraînement (9) est un électroaimant et/ou un moteur pas à pas (10a)
et l'alimentation électrique de l'unité d'entraînement (9)
est une source d'alimentation externe et/ou une batterie interne (16).

3. Instrument selon la revendication 1 ou 2, **caractérisé en ce que** un moteur pas à pas (10a) est relié au pied de support (6).

4. Instrument selon l'une quelconque des revendications 1 à 3, **caractérisé en ce que** la tête de compression (2) et/ou le mécanisme de fixation de libération (3) et/ou le boîtier (8) sont en acier inoxydable.

5. Instrument selon l'une quelconque des revendications 1 à 3, **caractérisé en ce que** il est équipé de trois plaques (5), et d'un système de mise à niveau automatique pour maintenir l'instrument (1) et les plaques (5) en position horizontale.

6. Procédé d'application de l'instrument selon la revendication 1, **caractérisé en ce que** le temps, la distance et la force de la mécanotransduction sont réglés sur l'ordinateur (12) et les données définies sont enregistrées ; la tête de compression (2) est déplacée par l'unité d'entraînement (9) ; la mesure est répétée, reproduite et vérifiée avec les données enregistrées.

7. Procédé selon la revendication 6, **caractérisé en ce que** pendant la mesure, l'instrument (1) doit être maintenu horizontalement.

8. Procédé selon la revendication 6 ou 7, **caractérisé en ce que** lors du remplacement de la tête de compression (2), le mécanisme de fixation de libération (3) est démonté puis remonté manuellement.

9. Procédé selon l'une quelconque des revendications 6 à 8, **caractérisé en ce que** il comprend également l'étape de stimulation de différentes cultures sur les trois plaques (5) avec trois programmes différents simultanément, avec des fréquences différentes, de manière mécaniquement reproductible.
